# EUROPÄISCHE PATENTSCHRIFT

(11) **EP 0 883 591 B1**
(45) Veröffentlichungstag und Bekanntmachung des Hinweises auf die Patenterteilung: **10.05.2000**
(21) Anmeldenummer: 97905123.2
(22) Anmeldetag: 28.02.1997
(51) Int. Cl.: C07C 29/149, C07C 31/20, C07D 313/04

(54) **VERFAHREN ZUR HERSTELLUNG VON 1,6-HEXANDIOL UND CAPROLACTON**
PROCESS FOR PREPARING 1,6 HEXANE DIOL AND CAPROLACTON
PROCEDE DE PRODUCTION DE 1,6 HEXANEDIOL ET DE CAPROLACTONE

(30) Priorität: 01.03.1996 DE 19607954; 15.11.1996 DE 19647349
(43) Veröffentlichungstag der Anmeldung: 16.12.1998
(73) Patentinhaber: BASF AKTIENGESELLSCHAFT, 67056 Ludwigshafen (DE)
(72) Erfinder: BAUR, Karl, Gerhard, D-67063 Ludwigshafen (DE); FISCHER, Rolf, D-69121 Heidelberg (DE); PINKOS, Rolf, D-67098 Bad Dürkheim (DE); STEIN, Frank, D-67098 Bad Dürkheim (DE); BREITSCHEIDEL, Boris, D-36043 Fulda (DE); RUST, Harald, D-67435 Neustadt (DE)
(86) Internationale Anmeldenummer: EP9700990
(87) Internationale Veröffentlichungsnummer: WO9731883

(56) Entgegenhaltungen:
- EP-A- 0 349 861
- EP-A- 0 661 255
- EP-A- 0 673 909
- DE-A- 1 618 143
- DE-A- 2 819 593

## Beschreibung

Die Erfindung betrifft ein Verfahren zur Herstellung von 1,6-Hexandiol und Caprolacton, bevorzugt mit mindestens 99 %iger Reinheit, die insbesondere von 1,4-Cyclohexandiolen praktisch frei sind, aus einem Carbonsäuregemisch, das als Nebenprodukt der Oxidation von Cyclohexan zu Cyclohexanon/Cyclohexanol mit Sauerstoff oder Sauerstoff enthaltenden Gasen und durch Wasserextraktion des Reaktionsgemisches erhalten wird, durch Veresterung und Hydrierung eines Teilstroms zu Hexandiol und Cyclisierung von 6-Hydroxycapronsäureester, wobei die 1,4-Cyclohexandiole entweder bei der Fraktionierung des Veresterungsgemisches oder zuletzt vom Caprolacton abgetrennt werden. Nach einer Abwandlung des Verfahrens der Erfindung wird die Hydrierung unterlassen und Adipinsäurediester gewonnen, der einer unmittelbaren Verwendung z.B. als Schmiermittel zugeführt werden kann.

1,6-Hexandiol stellt einen gesuchten Monomerbaustein dar, der überwiegend auf dem Polyester- und Polyurethansektor eingesetzt wird. Caprolacton bzw. die daraus durch Polyaddition hergestellten Polycaprolactone dienen zur Herstellung von Polyurethanen.

Die wäßrigen Lösungen von Carbonsäuren, die bei der Oxidation von Cyclohexan zu Cyclohexanol und Cyclohexanon (vgl. Ullmann's Encyclopedia of Industrial Chemistry, 5. Ed., 1987, Vol. A8, S. 49) als Nebenprodukte entstehen, im folgenden Dicarbonsäurelösung (DCL) genannt, enthalten (berechnet wasserfrei in Gew.-%) im allgemeinen zwischen 10 und 40 % Adipinsäure, zwischen 10 und 40 % 6-Hydroxycapronsäure, zwischen 1 und 10 % Glutarsäure, zwischen 1 und 10 % 5-Hydroxyvaleriansäure, zwischen 1 und 5 % 1,2-Cyclohexandiole, zwischen 1 und 5 % 1,4-Cyclohexandiole, zwischen 2 und 10 % Ameisensäure sowie eine Vielzahl weiterer Mono- und Dicarbonsäuren, Ester, Oxo- und Oxa-Verbindungen, deren Einzelgehalte im allgemeinen 5 % nicht übersteigen. Beispielsweise seien Essigsäure, Propionsäure, Buttersäure, Valeriansäure, Capronsäure, Oxalsäure, Malonsäure, Bernsteinsäure, 4-Hydroxybuttersäure und γ-Butyrolacton genannt.

Aus DE 2 321 101 und DE 1 235 879 ist bekannt, diese wäßrigen Dicarbonsäurelösungen bei Temperaturen von 120 bis 300°C und Drucken von 50 bis 700 bar in Gegenwart überwiegend Kobalt enthaltender Katalysatoren zu 1,6-Hexandiol als Hauptprodukt zu hydrieren. Die Hydrierausträge werden bevorzugt destillativ aufgearbeitet. Dabei gelingt es auch mit extrem hohem Destillationsaufwand nicht oder nur unvollständig, die bei der Hydrierung nicht veränderten 1,4-Cyclohexandiole von 1,6-Hexandiol zu trennen, so daß sich die 1,4-Cyclohexandiole, die anfänglich bereits in der DCL enthalten waren, mit einem Gehalt von im allgemeinen 2 bis 5 Gew.-% im 1,6-Hexandiol wiederfinden.

Um diesem Problem zu begegnen, sind einige Lösungsansätze bekannt:

In US 3 933 930 wird die Umsetzung von 1,4-Cyclohexandiol in wäßrigen Lösungen von Adipinsäure und 6-Hydroxycapronsäure zu Cyclohexanol, Cyclohexan und/oder Cyclohexen beschrieben, indem das Gemisch katalytisch vorhydriert wird. Dieses Verfahren bedarf des Einsatzes zweier verschiedener Hydrierkatalysatoren, eines für die Vorhydrierung, eines für die eigentliche Carbonsäurehydrierung und ist daher aufwendig.

Nach DE-OS 2 060 548 wird sehr reines 1,6-Hexandiol durch Kristallisation gewonnen. Auch dieses Verfahren ist sehr aufwendig und außerdem mit erheblichen Ausbeuteverlusten verbunden.

Eine weitere Möglichkeit zur Gewinnung von hochreinem 1,6-Hexandiol besteht darin, anstelle von DCL reine Adipinsäure oder reine Adipinsäureester zu hydrieren (K. Weissermel, H.J. Arpe, Industrielle Organische Chemie, VCH-Verlagsgemeinschaft Weinheim, 4. Auflage, Seite 263, 1994). Reine Adipinsäure ist jedoch im Vergleich zu DCL sehr teuer. Ferner ist das Carbonsäuregemisch, das bei der Cyclohexanoxidation anfällt, ein Abfallprodukt, das auch aus Umweltschutzgesichtspunkten einer stofflichen Verwertung zugeführt werden sollte.

Caprolacton wird bislang großtechnisch ausschließlich auf Basis Cyclohexanon durch Baeyer-Villiger-Oxidation hergestellt. Dabei werden explosive Perverbindungen entweder eingesetzt oder im Verfahren durchlaufen.

Auch die Herstellung von Caprolacton aus DCL ist z.B. aus DE 1 618 143 bereits beschrieben worden. Dabei wird entwässerte DCL mit Phosphorsäure thermisch umgesetzt und ein Gemisch aus Dicarbonsäuren, Caprolacton sowie eine Vielzahl anderer Komponenten fraktioniert. Der Sumpf fällt dabei z.T. fest und schwerlöslich an. Das Caprolacton hat aber auch nach weiterer destillativer Aufarbeitung nur eine 98 %ige Reinheit.

Ferner ist vielfach beschrieben, 6-Hydroxycapronsäure oder deren Ester zu Caprolacton umzusetzen (z.B. DE 2 013 525, EP-A 349 861 und darin zitierte Literatur).

Es bestand daher die Aufgabe, ausgehend von DCL die darin enthaltene 6-Hydroxycapronsäure in sehr reines Caprolacton umzuwandeln und gleichzeitig sehr reines 1,6-Hexandiol bzw. Adipinsäureester aus der in der DCL enthaltenen Adipinsäure zu gewinnen und damit die Nachteile des Standes der Technik, d.h. entweder hohe Kosten der Herstellung oder unzureichende Reinheit der Produkte, zu vermeiden.

Diese Aufgabe wurde gelöst mit einem Verfahren zur Herstellung von 1,6-Hexandiol und ε-Caprolacton aus einem Adipinsäure, 6-Hydrocapronsäure und geringe Mengen 1,4-Cyclohexandiole enthaltenden Carbonsäuregemisch, das als Nebenprodukt der Oxidation von Cyclohexan zu Cyclohexanon/Cyclohexanol mit Sauerstoff oder Sauerstoff enthaltenden Gasen und durch Wasserextraktion des Reaktionsgemisches erhalten wird, durch Veresterung und Hydrierung eines Teilstroms zu Hexandiol und Cyclisierung von 6-Hydroxycapronsäureester zu Caprolacton, wobei man
a) die in dem wäßrigen Reaktionsgemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in einen von 1,4-Cyclohexandiolen im wesentlichen freie Esterfraktion und eine zumindest den größeren Teil der 1,4-Cyclohexandiole enthaltende Fraktion durchführt,
d) von der Esterfraktion in einer dritten Destillationsstufe zumindest teilweise einen im wesentlichen 6-Hydroxycapronsäureester enthaltenden Strom abtrennt,
e) die Esterfraktion aus (d) aus der zumindest teilweise 6-Hydroxycapronsäureester entfernt wurde, katalytisch hydriert und durch Destillation des Hydrierproduktes in an sich bekannter Weise 1,6-Hexandiol gewinnt und
f) den im wesentlichen 6-Hydroxycapronsäureester enthaltenden Strom auf Temperaturen über 200°C bei vermindertem Druck erhitzt, wodurch 6-Hydroxycapronsäureester zu Caprolacton cyclisiert wird und aus dem Cyclisierungsprodukt durch Destillation reines ε-Caprolacton gewinnt.

Nach einer Abwandlung dieses Verfahrens kann auch der Adipinsäureester als solcher gewonnen und einer unmittelbaren Verwendung, z.B. als Schmiermittel zugeführt werden, d.h. die Stufe (e) kann weggelassen werden.

Es war überraschend, daß bei der Trennung der Estergemische, die durch die Veresterung der in der DCL enthaltenen Mono- und Dicarbonsäuren entstehen, die 1,4-Cyclohexandiole, die ja ebenfalls mit Carbonsäuren verestert vorliegen können, so abgetrennt werden können, daß nach Hydrierung und Aufarbeitung dem verbleibenden sehr geringen 1,4-Cyclohexandiolgehalt im 1,6-Hexandiol keine praktische Bedeutung mehr zukommt. Wegen der zu trennenden komplizierten Stoffgemische war es überraschend, daß es gelang, die 1,4-Cyclohexandiole oder deren Ester trotz der ungünstigen Siedepunktverhältnisse und zu befürchtenden Azeotropbildung, praktisch vollständig von den für die Hydrierung zu 1,6-Hexandiol verwendeten C₆-Estern abzutrennen.

Die Veresterung kann ohne Zusatz von Katalysatoren bevorzugt unter Einwirkung von Katalysatoren durchgeführt werden. Als niedermolekulare Alkohole kommen in der Regel solche mit 1 bis 10 C-Atomen, insbesondere Alkanole mit 1 bis 8 C-Atomen in Betracht. Auch Diole wie Butandiol oder Pentandiol kommen prinzipiell in Betracht. Soll Caprolacton und Adipinsäureester gewonnen werden, kommen auch Alkohole, die höher als Caprolacton sieden, wie z.B. 1,6-Hexandiol, Octadecanol oder Trimethylolpropan in Betracht.

Die technisch bevorzugten für die Veresterung zu verwendenden Alkohole sind n- oder i-Butanol und insbesondere Methanol.

Im Falle der Veresterung mit Methanol (Variante A) geht man so vor, daß man in der Destillationsstufe (c) eine im wesentlichen von 1,4-Cyclohexandiolen freie Carbonsäuremethylesterfraktion am Kopf der Kolonne und eine die Hochsieder und die 1,4-Cyclohexandiole enthaltende Sumpffraktion gewinnt und die Carbonsäuremethylesterfraktion in der Hydrierstufe (d) katalytisch hydriert.

Wird n- oder i-Butanol zur Veresterung verwendet (Variante B), werden in der Destillationsstufe (c) die 1,4-Cyclohexandiole mit den Leichtsiedern über Kopf abgetrennt und man gewinnt die Carbonsäurebutylester als Seitenabzug oder als diese enthaltenden Sumpf mit nachfolgender Einleitung in die Hydrierstufe (d), bzw. in eine weitere Destillation, falls man Adipinsäureester gewinnen will.

Das erfindungsgemäße Verfahren wird mit seinen Varianten A (Fig. 1), B (Fig. 2), C (Fig. 3) und D (Fig. 4) wie folgt allgemein erläutert (wobei die Begriffe über Kopf bzw. als Sumpf jeweils den Abzug oberhalb bzw. unterhalb des Zulaufs bedeuten):

### Variante A:

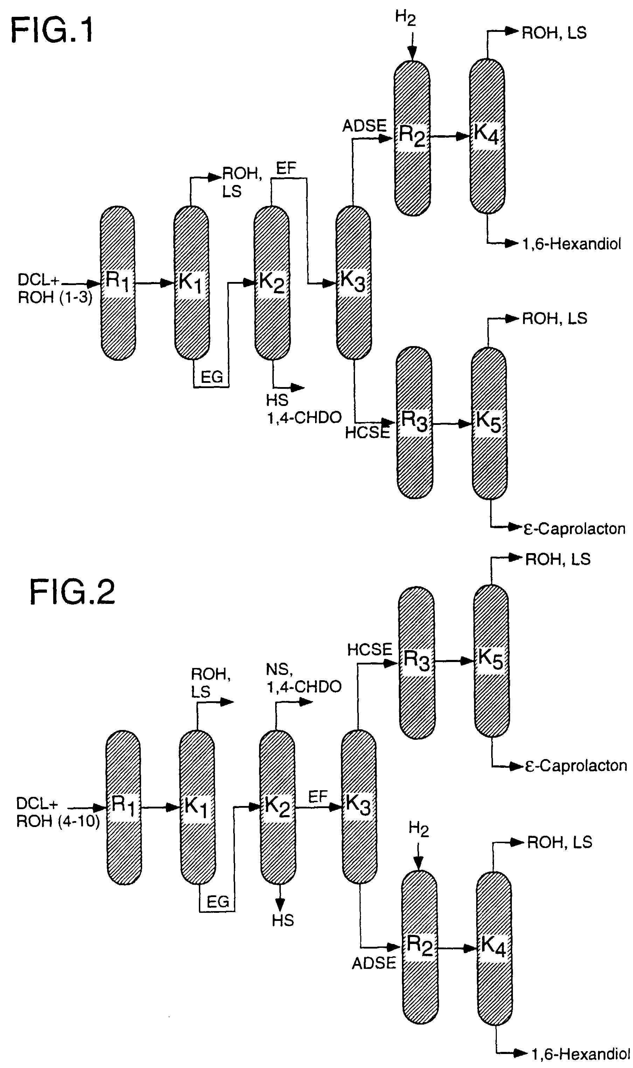

Wie in Fig. 1 dargestellt, wird die Dicarbonsäurelösung (DCL), gegebenenfalls nach Entwässerung, zusammen mit einem C₁- bis C₃-Alkohol, vorzugsweise Methanol, in den Veresterungsreaktor R₁ eingespeist, in dem die Carbonsäuren verestert werden. Das erhaltene Veresterungsgemisch gelangt dann in die Kolonne K₁, in der der überschüssige Alkohol (ROH), Wasser und Leichtsieder (LS) über Kopf abdestilliert und das Estergemisch (EG) als Sumpf abgezogen und in die Kolonne K₂ eingespeist wird. In dieser Kolonne wird das EG in eine im wesentlichen von 1,4-Cyclohexandiolen freie Esterfraktion (EF) und eine Sumpffraktion bestehend aus Hochsiedern (HS) und cis- und trans-1,4-Cyclohexandiolen (1,4-CHDO) fraktioniert. Die Esterfraktion wird dann in einen weitere Fraktionierkolonne K₃ geleitet, in der die Esterfraktion in ein Kopfprodukt, bestehend im wesentlichen aus Adipinsäurediester (ADSE), vorzugsweise -dimethylester und ein Sumpfprodukt bestehend im wesentlichen aus 6-Hydroxycapronsäureester (HCSE), vorzugsweise -methylester aufgetrennt wird.

Die im wesentlichen Adipinsäurediester enthaltende Fraktion wird dann in der katalytischen Hydrierung R₂ zu 1,6-Hexandiol hydriert, das in der Kolonne K₄ reindestilliert wird.

Die 6-Hydroxycapronsäureesterfraktion wird im Reaktor R₃ einer thermischen Behandlung über 100°C, in der Regel 150 bis 350°C, vorzugsweise 200 bis 300°C bei vermindertem Druck, z.B. 900 bis 10 mbar, vorzugsweise 300 bis 20 mbar unterworfen; dies führt zur Cyclisierung des Esters unter Bildung von ε-Caprolacton, das in der Kolonne K₅ reindestilliert wird.

### Variante B:

Sie unterscheidet sich von Variante A dadurch, daß man zur Veresterung Alkohole mit 4 bis 10 Kohlenstoffatomen verwendet. Vorzugsweise verwendet man n- oder i-Butanol. Man erhält dadurch in der Destillationskolonne durch die Erhöhung der Siedepunkte der Ester eine Umkehrung der Fraktionierung, d.h. die Esterfraktion (EF) fällt hier als Sumpfprodukt an. Wie in Fig. 2 dargestellt, wird dann das EF wiederum in die Fraktierkolonne K₃ geleitet, in der Adipinsäurediester, vorzugsweise der -dibutylester (ADSE) nunmehr als Sumpf und der 6-Hydroxycapronester, vorzugsweise -butylester, als Kopfprodukt erhalten wurde, worauf beide, wie in Variante A beschrieben, aufgearbeitet werden.

### Variante C:

Gemäß dieser Variante wird die Destillation der Kolonnen K₂ und K₃ zu einer Destillationsstufe zusammengezogen.

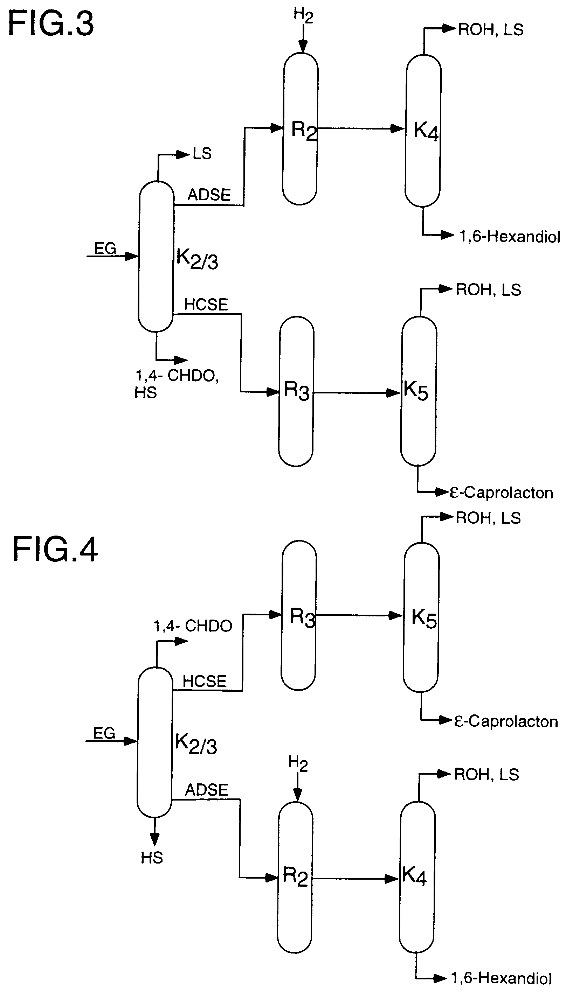

Gemäß Fig. 3 wird dabei das durch Veresterung mit Alkoholen mit 1 bis 3 C-Atomen, vorzugsweise Methanol, erhaltene Estergemisch (EG) einer fraktionierenden Destillation unterworfen und in einem oberen Seitenabzug der Adipinsäureester, vorzugsweise -dimethylester, in einem unteren Seitenabzug der 6-Hydroxycapronester, vorzugsweise -methylester, und als Sumpf die 1,4-Cyclohexandiole gewonnen.

Die Adipinsäureester- und 6-Hydroxycapronsäureesterfraktionen werden dann wie in Fig. 1 beschrieben aufgearbeitet.

### Variante D:

Diese Ausführungsform entspricht der Variante C mit dem Unterschied, daß zur Veresterung Alkohole einschließlich Diole mit 4 bis 10 Kohlenstoffatomen, vorzugsweise n- oder i-Butanol verwendet werden. Aufgrund der Umkehrung der Siedepunktrelationen erhält man als oberen Seitenabzug 6-Hydroxycapronsäureester als unteren Seitenabzug Adipinsäureester und als Kopfprodukt die 1,4-Cyclohexandiole. Die Weiterverarbeitung der Esterfraktionen erfolgt dann wie oben beschrieben.

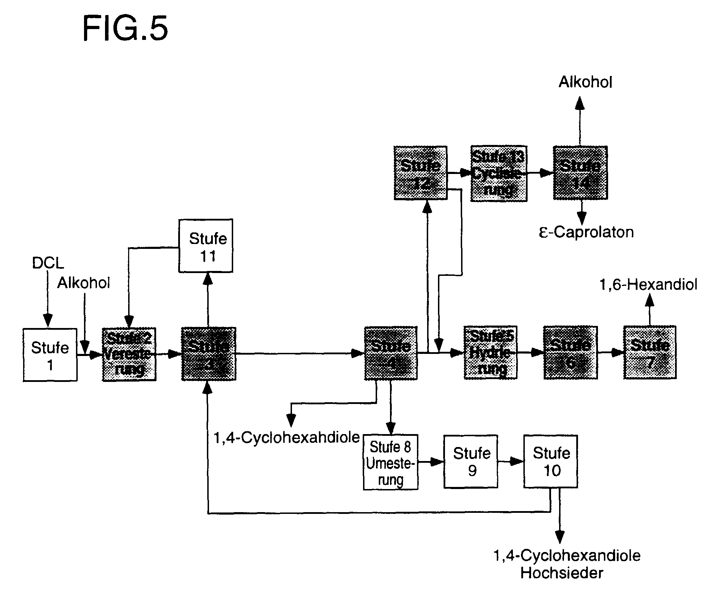

Das erfindungsgemäße Verfahren wird im folgenden für die Variante A im einzelnen anhand der Fig. 5 erläutert. Die Reaktionsbedingungen gelten für die anderen Varianten gleichermaßen.

Die Verfahrensschritte sind in Stufen aufgeschlüsselt, wobei die Stufen 2, 3, 4, 5, 6, 7 sowie 12, 13 und 14 für das Verfahren essentiell sind (die Stufen 5,6,7 allerdings nur, falls Hexandiol und nicht Adipinsäurediester als solcher gewonnen wird) und die Stufen 3 und 4 sowie 6 und 7 auch zusammengefaßt werden können. Die Stufen 8, 9, 10 und 11 sind fakultativ, aber zur Erhöhung der Wirtschaftlichkeit des Verfahrens gegebenenfalls sinnvoll.

Die Dicarbonsäurelösung (DCL) ist im allgemeinen eine wäßrige Lösung mit einem Wasseranteil von 20 bis 80 %. Da eine Veresterungsreaktion eine Gleichgewichtsreaktion darstellt, bei der Wasser entsteht, ist es sinnvoll, insbesondere bei Veresterung mit z.B. Methanol vorhandenes Wasser vor der Reaktion zu entfernen, vor allem wenn während der Veresterungsreaktion Wasser nicht, z.B. nicht azeotrop, entfernt werden kann. Die Entwässerung in Stufe 1 kann z.B. mit einem Membransystem erfolgen, oder bevorzugt durch eine Destillationsapparatur, bei der bei 10 bis 250°C, bevorzugt 20 bis 200°C, besonders 30 bis 200°C und einem Druck von 1 bis 1 500 mbar, bevorzugt 5 bis 1 100 mbar, besonders bevorzugt 20 bis 1 000 mbar Wasser über Kopf und höhere Monocarbonsäuren, Dicarbonsäuren und 1,4-Cyclohexandiole über Sumpf abgetrennt werden. Die Sumpftemperatur wird dabei bevorzugt so gewählt, daß das Sumpfprodukt flüssig abgezogen werden kann. Der Wassergehalt im Sumpf der Kolonne kann 0,01 bis 10 Gew.-%, bevorzugt 0,01 bis 5 Gew.-%, besonders bevorzugt 0,01 bis 1 Gew.-% betragen.

Die Abtrennung des Wassers kann so erfolgen, daß das Wasser überwiegend säurefrei erhalten wird oder man kann die in der DCL enthaltenen niederen Monocarbonsäuren - im wesentlichen Ameisensäure - zum größten Teil mit dem Wasser abdestillieren, damit diese in der Veresterung keinen Veresterungsalkohol binden.

Dem Carbonsäurestrom aus der Stufe 1 wird Alkohol ROH mit 1 bis 10 C-Atomen zugemischt. Dabei können Methanol, Ethanol, Propanol oder iso-Propanol oder Gemische der Alkohole, bevorzugt aber Methanol einerseits oder C₄ und höhere Alkohole, insbesondere mit 4 bis 8 C-Atomen und bevorzugt n- oder i-Butanol oder auch n-Pentanol oder i-Pentanol andererseits verwendet werden. Das Mischungsverhältnis Alkohol zu Carbonsäurestrom (Massenverhältnis) kann von 0,1 bis 30, bevorzugt 0,2 bis 20, besonders bevorzugt 0,5 bis 10 betragen.

Dieses Gemisch gelangt als Schmelze oder Lösung in den Reaktor der Stufe 2, in dem die Carbonsäuren mit dem Alkohol verestert werden. Die Veresterungsreaktion kann bei 50 bis 400°C, bevorzugt 70 bis 300°C, besonders bevorzugt 90 bis 200°C durchgeführt werden. Es kann ein äußerer Druck angelegt werden, bevorzugt wird die Veresterung aber unter Eigendruck des Reaktionssystems durchgeführt. Als Veresterungsapparat kann dabei ein Rührkessel oder Strömungsrohr oder es können jeweils mehrere verwendet werden. Die für die Veresterung notwendige Verweilzeit liegt zwischen 0,3 und 10 Stunden, bevorzugt 0,5 bis 5 Stunden. Die Veresterungsreaktion kann ohne Zusatz eines Katalysators ablaufen, bevorzugt wird aber zur Erhöhung der Reaktionsgeschwindigkeit ein Katalysator zugesetzt. Dabei kann es sich um einen homogenen gelösten oder um einen festen Katalysator handeln. Als homogene Katalysatoren seien beispielhaft Schwefelsäure, Phosphorsäure, Salzsäure, Sulfonsäuren wie p-Toluolsulfonsäure, Heteropolysäuren wie Wolframatophosphorsäure oder Lewissäuren wie z.B. Aluminium-, Vanadium-, Titan-, Bor-Verbindungen genannt. Bevorzugt sind Mineralsäuren, insbesondere Schwefelsäure. Das Gewichtsverhältnis von homogenem Katalysator zu Carbonsäureschmelze beträgt in der Regel 0,0001 bis 0,5, bevorzugt 0,001 bis 0,3.

Als feste Katalysatoren sind saure oder supersaure Materialien, z.B. saure und supersaure Metalloxide wie SiO₂, Al₂O₃, SnO₂, ZrO₂, Schichtsilikate oder Zeolithe, die alle zur Säureverstärkung mit Mineralsäureresten wie Sulfat oder Phosphat dotiert sein können, oder organische Ionentauscher mit Sulfonsäure-, oder Carbonsäuregruppen geeignet. Die festen Katalysatoren können als Festbett angeordnet oder als Suspension eingesetzt werden.

Das bei der Reaktion gebildete Wasser wird zweckmäßig kontinuierlich z.B. durch eine Membran oder destillativ entfernt.

Die Vollständigkeit des Umsatzes der in der Carbonsäureschmelze vorhandenen freien Carboxylgruppen wird mit der nach der Reaktion gemessenen Säurezahl (mg KOH/g) festgestellt. Sie beträgt abzüglich der gegebenenfalls zugesetzten Säure als Katalysator 0,01 bis 50, bevorzugt 0,1 bis 10. Dabei müssen nicht alle im System vorhandenen Carboxylgruppen als Ester des eingesetzten Alkohols vorliegen, sondern ein Teil kann in Form von dimeren oder oligomeren Estern mit dem OH-Ende der Hydroxycapronsäure vorliegen.

Das Veresterungsgemisch wird in Stufe 3, ein Membransystem oder bevorzugt eine Destillationskolonne, eingespeist. Wurde zur Veresterungsreaktion eine gelöste Säure als Katalysator eingesetzt, wird das Veresterungsgemisch zweckmäßig mit einer Base neutralisiert, wobei pro Säureäquivalent des Katalysators 1 bis 1,5 Basenäquivalente zugesetzt werden. Als Basen werden in der Regel Alkali- oder Erdalkalimetalloxide, -Carbonate, -Hydroxyde oder -Alkoholate, oder Amine in Substanz oder in dem Veresterungsalkohol gelöst verwendet.

Wird in Stufe 3 eine Kolonne verwendet, so erfolgt der Zulauf zur Kolonne bevorzugt zwischen dem Kopf- und dem Sumpfstrom. Über Kopf wird bei Drücken von 1 bis 1 500 mbar, bevorzugt 20 bis 1 000 mbar, besonders bevorzugt 40 bis 800 mbar und Temperaturen zwischen 0 und 150°C, bevorzugt 15 und 90°C und insbesondere 25 und 75°C der überschüssige Veresterungsalkohole ROH, Wasser sowie entsprechende Ester der Ameisensäure, Essigsäure und Propionsäure abgezogen. Dieser Strom kann entweder verbrannt oder bevorzugt in der Stufe 11 weiter aufgearbeitet werden.

Als Sumpf wird ein Estergemisch erhalten, das vorwiegend aus den Estern des eingesetzten Alkohols ROH mit Dicarbonsäuren wie Adipinsäure und Glutarsäure, Hydroxycarbonsäuren wie 6-Hydroxycapronsäure und 5-Hydroxyvaleriansäure, sowie aus Oligomeren und freien bzw. veresterten 1,4-Cyclohexandiolen besteht. Es kann sinnvoll sein, einen Restgehalt von Wasser und/oder Alkohol ROH bis je 4 Gew-% im Estergemisch zuzulassen. Die Sumpftemperaturen betragen 70 bis 250°C, bevorzugt 80 bis 220°C, besonders bevorzugt 100 bis 190°C.

Der weitgehend von Wasser und Veresterungsalkohol ROH befreite Strom aus Stufe 3 wird in die Stufe 4 eingespeist. Dabei handelt es sich um eine Destillationskolonne, bei der der Zulauf zwischen den leichtsiedenden Komponenten und den schwersiedenden Komponenten erfolgt. Die Kolonne wird bei Temperaturen von 10 bis 300°C, bevorzugt 20 bis 270°C, besonders bevorzugt 30 bis 250°C und Drucken von 1 bis 1 000 mbar, bevorzugt 5 bis 500 mbar, besonders bevorzugt 10 bis 200 mbar betrieben.

Die Kopffraktion besteht überwiegend aus Restwasser und Restalkohol ROH, Estern des Alkohols ROH mit Monocarbonsäuren, überwiegend C₃- bis C₆-Monocarbonsäureestern mit Hydroxycarbonsäuren, wie 6-Hydroxycapronsäure, 5-Hydroxyvaleriansäure sowie vor allem den Diestern mit Dicarbonsäuren wie Adipinsäure, Glutarsäure und Bernsteinsäure, 1,2-Cyclohexandiolen, Caprolacton und Valerolaceton.

Die genannten Komponenten können zusammen über Kopf abgetrennt oder in einer weiteren bevorzugten Ausführungsform in der Kolonne der Stufe 4 in einen Kopfstrom, der überwiegend Restwasser und Restalkohol sowie die oben erwähnten Bestandteile mit 3 bis 5 C-Atomen enthält und einen Seitenstrom, der überwiegend die oben erwähnten Bestandteile der C₆-Ester enthält, aufgetrennt werden. Der die Ester der C₆-Säuren enthaltende Strom, entweder als Gesamt-Kopfstrom oder als Seitenstrom kann dann, je nachdem wieviel Caprolacton hergestellt werden soll, im Grenzfall - ohne Caprolacton-Herstellung - ganz in die Hydrierung (Stufe 5) gelangen, erfindungsgemäß jedoch zum Teil oder als Gesamtstrom in die Stufe 12 eingespeist werden.

Die schwersiedenden Komponenten des Stromes aus Stufe 4, überwiegend bestehend aus 1,4-Cyclohexandiolen oder deren Estern, dimeren oder oligomeren Estern sowie nicht näher definierte z.T. polymeren Bestandteilen der DCL, werden über den Abtriebsteil der Kolonne der Stufe 4 abgetrennt. Diese können zusammen anfallen oder so, daß über einen Seitenstrom der Kolonne im Abtriebsteil vorwiegend die 1,4-Cyclohexandiole und über Sumpf der Rest abgetrennt werden. Die so gewonnenen 1,4-Cyclohexandiole können z.B. als Ausgangsstoff für Wirkstoffe Verwendung finden. Die schwersiedenden Komponenten, mit oder ohne den Gehalt an 1,4-Cyclodiolen, können entweder verbrannt werden oder in einer bevorzugten Ausführungsform zur sogenannten Umesterung in die Stufe 8 gelangen.

Die Stufen 3 und 4 können, insbesondere wenn nur kleinere Mengen verarbeitet werden, zusammengefaßt werden. Dazu kann beispielsweise in einer absatzweise durchgeführten fraktionierten Destillation der C₆-Esterstrom gewonnen werden, wiederum ohne daß 1,4-Cyclohexandiole in den zur Hydrierung geführten Strom gelangen.

Die Hydrierung erfolgt katalytisch entweder in der Gas- oder Flüssigphase. Als Katalysatoren kommen prinzipiell alle zur Hydrierung von Carbonylgruppen geeigneten homogenen und heterogenen Katalysatoren wie Metalle, Metalloxide, Metallverbindungen oder Gemische daraus in Betracht. Beispiele für homogene Katalysatoren sind in H. Kropf, Houben-Weyl, Methoden der Organischen Chemie, Band IV/lc, Georg Thieme Verlag Stuttgart, 1980, S. 45 bis 67, und Beispiele für heterogene Katalysatoren sind in Houben-Weyl, Methoden der Organischen Chemie, Band IV/lc, S. 16 bis 26, beschrieben.

Man verwendet bevorzugt Katalysatoren, die eines oder mehrere der Elemente aus den Nebengruppen I. und VI. bis VIII. des Periodensystems der Elemente, bevorzugt Kupfer, Chrom, Molybdän, Mangan, Rhenium, Ruthenium, Kobalt, Nickel und Palladium, besonders bevorzugt Kupfer, Kobalt oder Rhenium enthalten.

Die Katalysatoren können allein aus den Aktivkomponenten bestehen oder die Aktivkomponenten können auf Trägern aufgebracht sein. Als Trägermaterialien eignen sich z.B. Cr₂O₃, Al₂O₃, SiO₂, ZrO₂, TiO₂, ZnO₂, BaO und MgO oder Mischungen daraus.

Besonders bevorzugt sind Katalysatoren, wie sie in EP 0 552 463 beschrieben sind. Dies sind Katalysatoren, die in der oxidischen Form die Zusammensetzung

CuₐAl_{b}Zr_{c}Mn_{d}Oₓ

besitzen, wobei a > 0, b > 0, c ≙ 0, d > 0, a > b/2, b > a/4, a > c, a > d gilt und x die zur Wahrung der Elektroneutralität pro Formeleinheit erforderliche Anzahl von Sauerstoffionen bezeichnet. Die Herstellung dieser Katalysatoren kann beispielsweise nach den Angaben der EP 0 552 463 durch Fällung von schwerlöslichen Verbindungen aus Lösungen erfolgen, welche die entsprechenden Metallionen in Form ihrer Salze enthalten. Geeignete Salze sind beispielsweise Halogenide, Sulfate und Nitrate. Als Fällungsmittel eignen sich alle Agenzien, die zur Bildung solcher unlöslicher Zwischenstufen führen, die sich durch thermische Behandlung in die Oxide überführen lassen. Besonders geeignete Zwischenstufen sind die Hydroxide und Carbonate bzw. Hydrogencarbonate, so daß man als besonders bevorzugte Fällungsmittel Alkalicarbonate oder Ammoniumcarbonat einsetzt. Wichtig für die Herstellung der Katalysatoren ist die thermische Behandlung der Zwischenstufen bei Temperaturen zwischen 500°C und 1000°C. Die BET-Oberfläche der Katalysatoren liegt zwischen 10 und 150 m²/g.

Bevorzugt werden Heterogenkatalysatoren verwendet, die entweder fest angeordnet oder als Suspension eingesetzt werden. Wird die Hydrierung in der Gasphase und über fest angeordnetem Katalysator durchgeführt, werden im allgemeinen Temperaturen von 150 bis 300°C bei Drücken von 1 bis 100 bar, bevorzugt 15 bis 70 bar angewandt. Dabei wird zweckmäßig mindestens so viel Wasserstoff als Hydriermittel und Trägergas verwendet, daß Edukte, Zwischenprodukte und Produkte während der Reaktion nie flüssig werden. Der überschüssige Wasserstoff wird vorzugsweise im Kreis geführt, wobei ein kleiner Teil als Abgas zur Entfernung von Inerten wie z.B. Methan ausgeschleust werden kann. Es können dabei ein Reaktor oder mehrere Reaktoren hintereinander geschaltet verwendet werden.

Erfolgt die Hydrierung in der Flüssigphase mit fest angeordnetem oder suspendiertem Katalysator, so wird sie im allgemeinen bei Temperaturen zwischen 100 und 350°C, bevorzugt 120 und 300°C und Drücken von 30 bis 350 bar, bevorzugt 40 bis 300 bar durchgeführt.

Die Hydrierung kann in einem Reaktor oder mehreren hintereinander geschalteten Reaktoren durchgeführt werden. Die Hydrierung in Flüssigphase über einem Festbett kann man sowohl in Riesel- als auch Sumpffahrweise durchführen. Nach einer bevorzugten Ausführungsform verwendet man mehrere Reaktoren, wobei im ersten Reaktor der überwiegende Teil der Ester hydriert wird und der erste Reaktor bevorzugt mit Flüssigkeitskreislauf zur Wärmeabfuhr und der oder die nachfolgenden Reaktoren bevorzugt ohne Umlauf zur Vervollständigung des Umsatzes betrieben werden.

Die Hydrierung kann diskontinuierlich, bevorzugt kontinuierlich erfolgen.

Der Hydrieraustrag besteht im wesentlichen aus 1,6-Hexandiol und dem Alkohol ROH. Weitere Bestandteile sind vor allem, falls der gesamte leichtsiedende Strom der Stufe 4 eingesetzt wurde, 1,5-Pentandiol, 1,4-Butandiol, 1,2-Cyclohexandiole sowie kleine Mengen von Monoalkoholen mit 1 bis 6 C-Atomen und Wasser.

Der Hydrieraustrag wird in der Stufe 6, z.B. ein Membransystem oder bevorzugt eine Destillationskolonne, in den Alkohol ROH, der zusätzlich den größten Teil der weiteren leichtsiedenden Komponenten enthält und einen Strom, der überwiegend 1,6-Hexandiol neben 1,5-Pentandiol und den 1,2-Cyclohexandiolen enthält, aufgetrennt. Dabei werden bei einem Druck von 10 bis 1 500 mbar, bevorzugt 30 bis 1 200 mbar, besonders bevorzugt 50 bis 1 000 mbar Kopftemperaturen von 0 bis 120°C, bevorzugt 20 bis 100°C, besonders bevorzugt 30 bis 90°C sowie Sumpftemperaturen von 100 bis 270°C, bevorzugt 140 bis 260°C, besonders bevorzugt 160 bis 250°C eingestellt. Der leichtsiedende Stoffstrom kann entweder direkt in die Veresterung der Stufe 2 zurückgeführt werden oder in die Stufe 8 oder in die Stufe 11 gelangen.

Der 1,6-Hexandiol enthaltene Stoffstrom wird in der Stufe 7 in einer Kolonne gereinigt. Dabei werden 1,5-Pentandiol, die 1,2-Cyclohexandiole sowie weitere eventuell vorhandene Leichtsieder über Kopf abgetrennt. Sollen die 1,2-Cyclohexandiole und/ oder 1,5-Pentandiol als zusätzliche Wertprodukte gewonnen werden, so können diese in einer weiteren Kolonne aufgetrennt werden. Über den Sumpf werden eventuell vorhandene Hochsieder ausgeschleust. 1,6-Hexandiol wird mit einer Reinheit von mindestens 99 % aus einem Seitenstrom der Kolonne entnommen. Dabei werden bei Drücken von 1 bis 1 000 bar, bevorzugt 5 bis 800 mbar, besonders bevorzugt 20 bis 500 mbar Kopftemperaturen von 50 bis 200°C, bevorzugt 60 bis 150°C und Sumpftemperaturen von 130 bis 270°C, bevorzugt 150 bis 250°C eingestellt.

Sollen nur kleinere Mengen 1,6-Hexandiol hergestellt werden, so können die Stufen 6 und 7 auch in einer diskontinuierlichen fraktionierten Destillation zusammengefaßt werden.

Um das erfindungsgemäße Verfahren möglichst wirtschaftlich zu betreiben, ist es sinnvoll, den Veresterungsalkohol ROH zurückzugewinnen und immer wieder zur Veresterung einzusetzen. Dazu kann der vorwiegend den Alkohol ROH enthaltende Strom aus Stufe 3 und/ oder 6 in der Stufe 11 aufgearbeitet werden. Dazu wird vorteilhaft eine Kolonne verwendet, in der Komponenten, die leichter sieden als der Alkohol ROH über Kopf, Wasser und Komponenten, die höher sieden als der Alkohol ROH, über Sumpf vom Alkohol ROH, der in einem Seitenstrom gewonnen wird, abgetrennt werden. Die Kolonne wird zweckmäßig bei 500 bis 5 000 mbar, bevorzugt bei 800 bis 3 000 mbar betrieben.

Nach einer weiteren bevorzugten Ausführungsform des erfindungsgemäßen Verfahrens wird der hochsiedende Strom aus Stufe 4 zur Erhöhung der Gesamtausbeute an Wertprodukten, bezogen auf eingesetzte DCL aufgearbeitet. Dazu wird in der Stufe 8 der Anteil an dimeren und oligomeren Estern der Adipinsäure bzw. Hydroxycapronsäure mit weiteren Mengen des Alkohols ROH, vorzugsweise Methanol, in Gegenwart eines Katalysators umgesetzt. Das Gewichtsverhältnis von Alkohol ROH und dem Sumpfstrom aus Stufe 4 beträgt zwischen 0,1 bis 20, bevorzugt 0,5 bis 10, besonders bevorzugt 1 bis 5. Als Katalysatoren eignen sich prinzipiell die bereits für die Veresterung in Stufe 2 beschrieben. Bevorzugt werden jedoch Lewissäuren eingesetzt. Beispiele hierzu sind Verbindungen oder Komplexe des Aluminiums, Zinns, Antimons, Zirkons oder Titans, wie Zirkoniumacetylacetonat oder Tetraalkyltitanat wie Tetraisopropyltitanat, die in Konzentrationen von 1 bis 10 000 ppm, bevorzugt 50 bis 6 000 ppm, besonders bevorzugt 100 bis 4 000 ppm, angewandt werden. Besonders bevorzugt sind Titanverbindungen.

Die Umesterung kann absatzweise oder kontinuierlich, in einem Reaktor oder mehreren Reaktoren, in Reihe geschaltenen Rührkesseln oder Rohrreaktoren bei Temperaturen zwischen 100 und 300°C, bevorzugt 120 bis 270°C, besonders bevorzugt 140 bis 240°C und den sich dabei einstellenden Eigendrücken, durchgeführt werden. Die benötigten Verweilzeiten liegen bei 0,5 bis 10 Stunden, bevorzugt bei 1 bis 4 Stunden.

Dieser Strom aus der Stufe 8 läßt sich im Falle der Veresterung mit Methanol z.B. wieder in die Stufe 3 einschleusen. Zur Vermeidung von Aufpegelungen, vor allem von l,4-Cyclohexandiolen, muß dann absatzweise oder kontinuierlich ein Teilstrom der Hochsieder aus Stufe 4 ausgeschleust werden. Eine andere Möglichkeit ist, den Strom aus Stufe 8 nicht in Stufe 3 zurückzuführen, sondern ihn, analog zur Stufe 3, in einer Stufe 9 in vorwiegend Alkohol ROH, der dann wieder in die Stufe 2, 8 oder 11 gelangen kann und einen Strom der die Ester enthält, aufzutrennen.

Dieser Esterstrom kann prinzipiell (mit der Maßgabe der Vermeidung von Aufpegelungen der 1,4-Cyclohexandiole) in die Stufe 4 zurückgeführt werden oder wird bevorzugt in eine weitere Stufe 10 in die Ester der C₆-Säuren und, mengenmäßig eher unbedeutend, in die Ester der C₅-Säuren einerseits, die entweder in die Stufe 4 oder direkt in die Stufe 5 eingeschleust werden und Hochsieder andererseits, die 1,4-Cyclohexandiole enthalten, aufgetrennt, worauf die Hochsieder ausgeschleust werden.

Für die Caprolactonherstellung wird der vorwiegend Ester der C₆-Säuren enthaltende Strom aus der Stufe 4 eingesetzt. Dazu wird dieser Strom in Stufe 12, einer Destillationskolonne, in einen überwiegend Adipinsäurediester enthaltenden Strom, der die vorhandenen 1,2-Cyclohexandiole enthält, über Kopf und einen überwiegend 6-Hydroxycapronsäureester enthaltenden Strom, der keine 1,2-Cyclohexandiole enthält, über Sumpf aufgetrennt. Die Kolonne wird bei Drücken von 1 bis 500 mbar, bevorzugt 5 bis 350 mbar, besonders bevorzugt 10 bis 200 mbar und Sumpftemperaturen von 80 bis 250°C, bevorzugt 100 bis 200°C, besonders bevorzugt 110 bis 180°C betrieben. Die Kopftemperaturen stellen sich dabei entsprechend ein.

Wichtig für eine hohe Reinheit und hohe Ausbeute an Caprolacton ist die Abtrennung der 1,2-Cyclohexandiole vom Hydroxycapronsäureester, da diese Komponenten Azeotrope miteinander bilden. Es war in dieser Stufe 12 nicht vorauszusehen, daß die Trennung der 1,2-Cyclohexandiole und des Hydroxycapronsäureesters vollständig gelingt, vor allem wenn als Ester der bevorzugte Methylester eingesetzt wird.

Zur Verminderung des Trennaufwands kann es vorteilhaft sein in der Stufe 12 zusammen mit dem Adipinsäurediester auch etwas Hydroxycapronsäureester abzutrennen. Die Gehalte an Hydroxycapronsäureester liegen dabei vorteilhaft zwischen 0,2 und 7 Gew.-%. Je nach Alkoholkomponente der Ester wird dieser Anteil Hydroxycapronsäureester zusammen mit dem Adipinsäurediester über Kopf (z.B. Methylester) oder über Sumpf (z.B. Butylester) abgetrennt.

Der 6-Hydroxycapronsäureester enthaltende Sumpfstrom der Stufe 12 wird in der Stufe 13 in an sich bekannter Weise entweder in der Gas- oder Flüssigphase zu Alkohol und Caprolacton umgesetzt. Bevorzugt ist die Flüssigphase.

Die Reaktion wird ohne Katalysator oder aber bevorzugt in Anwesenheit eines Katalysators durchgeführt. Als Katalysatoren eignen sich saure oder basische Katalysatoren, die homogen gelöst oder heterogen vorliegen können. Beispiele sind Alkali- und Erdalkalimetallhydroxide-, -oxide, -carbonate, -alkoxylate oder -carboxylate, Säuren wie Schwefel- oder Phosphorsäure, organische Säuren wie Sulfonsäuren oder Mono- oder Dicarbonsäuren, bzw. Salze der vorgenannten Säuren, Lewissäuren, bevorzugt aus der III. und IV. Hauptgruppe bzw. der I. bis VIII. Nebengruppe des Periodensystems der Elemente.

Bevorzugt verwendet man die gleichen Katalysatoren, die auch in der Stufe 8 eingesetzt werden, da der hochsiedende Ausschleusstrom der Stufe 13 oligomere Hydroxycapronsäureeinheiten enthält, die vorteilhaft über die Stufe 8 wiederverwertet werden können. Wird ein heterogener Katalysator eingesetzt, so beträgt die Katalysatorbelastung üblicherweise 0,05 bis 5 kg Edukt/l Katalysator und Stunde. Bei homogenen Katalysatoren wird der Katalysator bevorzugt dem Eduktstrom zugemischt. Dabei beträgt die Konzentration üblicherweise 10 bis 10000 ppm, bevorzugt 50 bis 5000 ppm, besonders bevorzugt 100 bis 1000 ppm. Die Reaktion wird üblicherweise bei 150 bis 400°C, bevorzugt 180 bis 350°C, besonders bevorzugt 190 bis 330°C und Drücken von 1 bis 1020 mbar, bevorzugt 5 bis 500 mbar, besonders bevorzugt 10 bis 200 mbar durchgeführt.

In manchen Fällen ist es vorteilhaft die Cyclisierungsreaktion in Gegenwart von hochsiedenden Mono-, Di- oder Polyolen wie z.B. Decanol, Undecanol, Tridecanol, Pentadecanol, Octadecanol, 1,4-Butandiol, 1,5-Pentandiol, 1,6-Hexandiol, 1,4-Cyclohexandiolen, Butylethylpropandiol, Neopentylglycol, Triethylenglykol, Tetraethylenglykol, Trimethylolpropan oder Glycerin durchführen.

Diese hochsiedenden Alkohole oder Polyole werden vorgelegt und/ oder dem Reaktionsgemisch in Konzentrationen bis 5 Gew.-% zugesetzt oder separat zudosiert z.B. jeweils in Konzentrationen von 1 und 20000 ppm, bevorzugt 10 bis 4000 ppm, besonders bevorzugt 50 bis 2000 ppm.

Wird die Cyclisierung in der Flüssigphase durchgeführt, werden die Reaktionsprodukte, vorwiegend Veresterungsalkohol ROH und Caprolacton gasförmig aus dem Reaktionsgemisch entfernt. Vorteilhaft ist eine dem Reaktionsgefäß aufgesetzte Kolonne, in der noch nicht umgesetztes Edukt im Reaktionssystem gehalten werden kann und über Kopf der Alkohol und Caprolacton abgezogen werden. Dabei kann die Kondensation des Produktstroms so erfolgen, daß fraktioniert kondensiert wird, d.h. zuerst vorwiegend Caprolacton, dann der Veresterungsalkohol. Selbstverständlich kann auch nur der Alkohol über Kopf, Caprolacton dagegen in einem Seitenstrom gewonnen werden. Der Alkohol-Strom kann vorteilhaft in die Stufe 2, 8 oder 11 zurückgeführt werden. Das Sumpfprodukt der Cyclierung kann in die Stufe 8 eingeschleust werden.

Der Zulauf zum Reaktionsgefäß kann ohne Vorheizung erfolgen. Werden homogene Katalysatoren verwendet, so ist es vorteilhaft, den Eduktstrom direkt in den Cyclisierungssumpf einzutragen. Dabei kann der Katalysator entweder schon vor der Reaktion dem Feed zugefügt werden, oder direkt in das Reaktionsgefäß gegeben werden.

Vorteilhafter ist es jedoch den Zulauf vorzuheizen, vor allem, wenn der Katalysator bereits gelöst ist und ein Hydroxycapronsäureester mit einer C₁-C₅-Alkoholkomponente verwendet wird. Die Vorheiztemperatur liegt dabei zwischen 100 und 300°C, bevorzugt bei 130 - 270°C, besonders bevorzugt bei 150 - 250°C. Bei diesen Temperaturen reagiert der Hydroxycapronsäureester bereits zum Teil zu Alkohol, Caprolacton und dimeren bzw. oligomeren Hydroxycapronsäureestern. Dies bewirkt, daß nur wenig Hydroxycapronsäureester, wenn er in das heiße Reaktionsgefäß gelangt, sofort aus dem Reaktionssumpf abdestillieren kann. Auf diese Weise werden Kolonnenböden eingespart.

Eine weitere vorteilhafte Möglichkeit besteht darin, den überwiegenden Teil des Esteralkohols vor der Aufarbeitung des Caprolactons zu gewinnen, vor allem wenn dieser Alkohol, wie Methanol, niedrig siedet und in der Folge nur aufwendig kondensierbar wäre. Dazu wird der Hydroxycapronsäuremethylester in Gegenwart eines Katalysators wie oben beschrieben vorerhitzt, wobei bereits der freiwerdende Alkohol abdestilliert. Dies geschieht vorteilhaft bei 100-1100 mbar, einem Druckbereich, bei dem der Esteralkohol leicht kondensierbar ist. Dieses Vorgehen ist auch in Gegenwart der oben beschriebenen hochsiedenden Alkohole möglich.

Der Caprolacton-Produktstrom der Stufe 13 wird in der Stufe 14 weiter aufgearbeitet. Dabei kann es sich um eine oder mehrere Kolonnen handeln. Wird eine Kolonne verwendet, so werden über Kopf der gegebenenfalls noch vorhandene Veresterungsalkohol, sowie andere C₁- bis C₆-Leichtsieder abgetrennt, über Seitenstrom reines Caprolacton und über den Sumpf gegebenenfalls noch nicht umgesetzter Hydroxycapronsäureester, der zurückgeführt wird.

Hochreines Caprolacton erhält man, wenn in der Stufe 14 die erwähnten Leichtsieder in einer ersten Kolonne über Kopf, Caprolacton und andere Hochsieder über Sumpf in eine zweite Kolonne eingespeist werden, wo Caprolacton über Kopf abgezogen wird. Handelt es sich bei dem zu gewinnenden Caprolactonstrom nur um kleinere Mengen, so kann Caprolacton mit einer Kolonne durch absatzweise fraktionierte Destillation gewonnen werden.

Die Destillationen werden bei Sumpftemperaturen von 70 bis 250°C, bevorzugt 90 bis 230°C, besonders bevorzugt 100 bis 210°C und Drücken von 1 bis 500 mbar, bevorzugt 5 bis 200 mbar, besonders bevorzugt 10 bis 150 mbar durchgeführt.

Nach einer weiteren Abwandlung des Verfahrens - im folgenden Variante E - ist es möglich, insbesondere kleinere Mengen von Caprolacton mit minimalem technischen Aufwand nach einem Batchverfahren herzustellen. Dazu verwendet man für die Veresterung der Stufe (a) einen Alkohol, der höher als Caprolacton siedet und führt die Umsetzung ohne Isolierung der Adipinsäureesterfraktion ansatzweise in einer Eintopfreaktion in Gegenwart eines Veresterungskatalysators durch, wobei die Umsetzungen der Stufen (b), (c) und (f) im gleichen Ansatz erfolgen, die hochsiedenden Adipinsäureester im Sumpf verbleiben und Caprolacton als Destillat gewonnen wird.

Bei dieser Variante E werden zur Veresterung z.B. Alkohole wie Undecanol, Tridecanol, Pentadecanol, Octadecanol, 1,6-Hexandiol, 1,4-Cyclohexandiole, Butylethylpropandiol, Neopentylglycol, Triethylenglycol, Tetraethylenglycol, Trimethylolpropan oder Glycerin verwendet. Die Veresterung kann im Vakuum bei Temperaturen durchgeführt werden, bei denen einerseits Reaktionswasser, andererseits auch Komponenten wie die 1,2-Cyclohexandiole abdestillieren. Diese können natürlich auch erst nach der Veresterung destillativ abgetrennt werden. Der Veresterungsaustrag wird mit weiterem Katalysator wie oben beschrieben versetzt und bei 1 - 200 mbar, bevorzugt 10 - 50 mbar unter Destillationsbedingungen erhitzt. Dabei können Leichtsieder, z.B. die 1,2-Cyclohexandiole, abdestilliert werden, bevor Caprolacton durch Cyclisierung im Sumpf entsteht. Die bevorzugte Cyclisierungstemperatur liegt zwischen 200 und 300°C. Die Aufarbeitung des Caprolactons erfolgt wie oben beschrieben in einer oder mehreren Reindestillationskolonnen.

Nach dem erfindungsgemäßen Verfahren können Ausbeuten an 1,6-Hexandiol und Caprolacton von jeweils über 95 %, bei Reinheiten von über 99 % erzielt werden.

Unter Auslassung der Hydrierstufe gewonnene Adipinsäurediester können als Einsatzstoff auf dem Schmierstoffsektor, als Edukt für Cyclopentanon oder als Weichmacher dienen.

Das Verfahren wird anhand der nachfolgenden Beispiele näher erläutert.

### Beispiel 1 (Variante A)

### Stufe 1: (Entwässerung)

0,1 kg Dicarbonsäurelösung/h (Adipinsäure, 6-Hydroxycapronsäure, 1,4-Cyclohexandiole, Glutarsäure, 5-Hydroxyvaleriansäure, Ameisensäure, Wasser) wurden kontinuierlich in einer Destillationsapparatur (dreibödige Glockenbodenkolonne mit außenliegendem Öl-Heizkreislauf, Öltemperatur 150°C, Bodenvolumen je ca. 25 ml, Zulauf über den Glockenböden) mit aufgesetzter Füllkörperkolonne (ca. 4 theoretische Trennstufen, kein Rücklauf am Kopf) destilliert. Als Kopfprodukt wurden 0,045 kg erhalten mit einem Ameisensäuregehalt im Wasser von ca. 3 %. Im Sumpfstrom (5,5 kg) betrug der Wassergehalt ca. 0,4 %.

### Stufe 2: (Veresterung)

5,5 kg des Sumpfstroms aus Stufe 1 wurde mit 8,3 kg Methanol und 14 g Schwefelsäure umgesetzt. Die Säurezahl des Austrags abzüglich Schwefelsäure betrug ca. 10 mg KOH/g.

### Stufe 3:

In einer Kolonne wurden der Veresterungsstrom aus Stufe 2 destilliert (1015 mbar, 65°C Kopftemperatur, bis 125°C Sumpftemperatur). Über Kopf wurden 7,0 kg abgezogen. Als Sumpfprodukt wurden 6,8 kg erhalten.

### Stufe 4: (1,4-Cyclohexandiolabtrennung)

In einer 50 cm Füllkörperkolonne wurde der Sumpfstrom aus Stufe 3 fraktioniert destilliert (1 mbar, 70-90°C Kopftemperatur, bis 180°C Sumpftemperatur). Im Sumpf fanden sich die 1,4-Cyclohexandiole.

Als Leichsieder wurden 0,6 kg abdestilliert (1,2-Cyclohexandiole, Valerolacton, 5-Hydroxyvaleriansäuremethylester, Glutarsäuredimethylester, Bernsteinsäuredimethylester u.a.); als überwiegend Adipinsäuredimethylester und 6-Hydroxycapronsäuremethylester enthaltende Fraktion wurden 4,3 kg erhalten.

### Stufe 5: (Hydrierung Teilstrom; alternativ Abzug des C₆-Estergemisches und Reindestillation zur Gewinnung von Adipinsäuredimethylester)

2,7 kg C₆-Estergemisch aus Stufe 4 wurden kontinuierlich in einem 25 ml Reaktor an einem Katalysator hydriert (Katalysator, 70 Gew.-%, CuO, 25 Gew.-% ZnO, 5 Gew.-% Al₂O_{3,} der zuvor im Wasserstoffstrom bei 180°C aktiviert worden ist, Hydrierbedingungen: Zulauf 20 g/h, keim Umlauf, 220 bar, 220°C). Der Ester-Umsatz betrug 99,5 %, die 1,6-Hexandiolselektivität betrug über 99 %.

Unter Verwendung eines anderen Katalysators wurde die Esterfraktion in einer zweistufigen Reaktorkaskade (1. Reaktor 2,5 1 Katalysator, Rieselfahrweise, 250 bar, Produktrückführung : Zulauf = 10 : 1, 220 - 230°C; 2. Reaktor 0,5 l Katalysator, Rieselfahrweise gerader Durchgang, 260 bar, 220°C) kontin. hydriert. Als Katalysator wurde ein zuvor bei 180°C aktivierter Katalysator aus CuO (60 %), Al₂O₃ (30 %) und Mn₂O₃ (10 %) eingesetzt. Die Zulaufmenge betrug 1 kg/h. Bei 99,5 % Umsatz betrug die Hexandiol-Selektivität über 99 %.

### Stufe 6 und 7: (Hexandiolreinigung)

2,5 kg des Hydrieraustrags aus Stufe 5 wurden fraktioniert destilliert (Destillationsblase mit aufgesetzter 70 cm Füllkörperkolonne, Rücklaufverhältnis 2). Bei 1013 mbar wurden 0,5 kg Methanol abdestilliert und nach Anlegen von Vakuum (20 mbar) destillierten überwiegend die 1,2-Cyclohexandiole und 1,5-Pentandiol ab. Danach (Sdp. 146°C) destillierte 1,6-Hexandiol mit einer Reinheit von 99,8 % ab.

### Stufe 8:

2,9 kg des Sumpfaustrages der Stufe 4 wurden mit 3,8 kg Methanol und 3,8 g Tetra-i-propyltitanat versetzt und kontinuierlich in einem 1 m langen, 440 ml fassenden Rohrreaktor, der mit 3 mm V2A-Ringen gefüllt war, umgesetzt. Die mittlere Verweilzeit betrug ca. 2 h.

### Stufe 9:

Der Austrag aus Stufe 8 wurde analog der in Stufe 3 beschriebenen Apparatur fraktioniert destilliert. Bei 65°C Kopftemperatur wurden 3,5 kg abdestilliert (überwiegend Methanol). Im Sumpf verblieben 2,2 kg.

### Stufe 10:

Der Sumpf aus Stufe 9 wurde analog Stufe 4 bis zu einer Sumpftemperatur von 160°C fraktioniert destilliert. Als Destillat wurden 1,3 kg erhalten, das direkt hydriert oder in die 4. Stufe zurückgeführt werden kann. (Zusammensetzung: 52 % 6-Hydroxycapronsäuremethylester, 31 % Adipinsäuredimethylester, 5 % Glutarsäuredimethylester, 4 % 5-Hydroxycapronsäuremethylester sowie eine Vielzahl weiterer, mengenmäßig unbedeutender Komponenten).

### Stufe 11:

7 kg des Kopfproduktes der Stufe 3 wurden an einer 20 cm Füllkörperkolonne bei 1015 mbar fraktioniert destilliert. Es wurden 0,8 kg Vorlauffraktion bei 59 - 65°C Kopftemperatur erhalten, die neben vorwiegend Methanol, C₁- C₄-Monoethylester enthielt. Bei 65°C Kopftemperatur wurden 5,6 kg Methanol mit einer Reinheit > 99 % erhalten. Der Sumpf (0,6 kg) bestand überwiegend aus Wasser.

### Stufe 12:

Von 1,6 kg Estergemisch aus Stufe 4 wurden in einer 2 1 Destillationsblase mit aufgesetzter Kolonne (40 cm, 5 mm V2A-Metallringfüllkörper) und Rücklaufteiler bei 2 mbar vorwiegend Adipinsäuredimethylester abdestilliert (Rücklaufverhältnis 2, Kopftemperatur bis 91°C, Sumpftemperatur bis 118°C). Im Sumpf blieben 0,31 kg Hydroxycapronsäuremethylester (82 %ig, Rest vorwiegend dimerer Hydroxycapronsäuremethylester, kein Adipinsäuredimethylester) zurück.

### Stufe 13: (Cyclisierung)

In einer 250 ml Destillationsblase mit außenliegender Heizung und aufgesetzter Kolonne (70 cm, 5 mm V2A-Metallringfüllkörper) mit Rücklaufteiler wurden 60 ml Sumpfprodukt aus Stufe 12 mit Zusatz von 1000 ppm Tetraisopropyltitanat vorgelegt, auf 260°C bei 40 mbar aufgeheizt und stündlich 35 ml Sumpfprodukt aus Stufe 12, dem 1000 ppm Tetraisopropyltitanat sowie 200 ppm 1,6-Hexandiol zugesetzt wurden, zugefahren. Bei einer Kopftemperatur von 123 bis 124°C und einem Rücklaufverhältnis von 4 wurden bei 25°C vorwiegend Caprolacton, bei -78°C Methanol kondensiert.

### Stufe 14: (Caprolactonreinigung)

In einer 250 ml Destillationsblase mit aufgesetzter Kolonne (70 cm, 5 mm V2A-Metallringfüllkörper) und Rücklaufteiler (Rücklaufverhältnis 4) wurde das aus Stufe 13 erhaltene Caprolacton fraktioniert bei 40 mbar destilliert. Nach Abtrennung von im wesentlichen Valerolacton (Sdp. 90 bis 110°C) wurde Caprolacton (Sdp. 131°C) in einer Reinheit (GC-Flächen-%) von 99,9 % erhalten.

### Beispiel 2:

Analog Beispiel 1 wurde die Stufe 13 (Cyclisierung) mit einem Hydroxycapronsäure-haltigen Strom folgender Zusammensetzung betrieben: 81 % Hydroxycapronsäuremethylester, 9 % dimerer Hydroxycapronsäuremethylester, 0,1 % Adipinsäuredimethylester sowie Ester aus 5-Hydroxyvaleriansäuremethylester und Hydroxycapronsäuremethylester und oligomere Hydroxycapronsäuremethylester. Dabei wurden in dem 250 ml Reaktionsgefäß 50 g 1,6-Hexandiol, 30 g des Hydroxycapronsäuremethylester-haltigen Stroms und 0,1 g Titanat vorgelegt, die Mischung bei 40 mbar erhitzt und ab 155°C Sumpftemperatur kontinuierlich 30 g/h Esterzulauf (enthielt 1000 ppm Titanat) zugegeben. Bei Sumpftemperaturen zwischen 205 und 260°C wurden innerhalb 56 h 1225 g Caprolacton-haltiges Destillat, 336 g Methanol-haltiges Kühlfallenprodukt und ca. 100 g Sumpfprodukt erhalten. Der zu 100 % fehlende Rest geht auf Methanolverluste und den hold-up der Kolonne zurück. Nach fraktionierter Destillation des Caprolacton-haltigen Austrags wie in Beispiel 1 wurde Caprolacton mit Reinheiten zwischen 99 und 99,9 % erhalten.

### Beispiel 3 (Variante B):

2400 g Dicarbonsäurelösung gemäß Beispiel 1, 2400 g n-Butanol und 6 g Schwefelsäure wurden in einem Reaktionsgefäß mit aufgesetzter 20 cm Füllkörperkolonne mit Wasserabscheider so lange unter Rückfluß erhitzt, bis kein Wasser mehr überging (ca. 8 Stunden). Dabei wurde das heterogene Butanol/Wasser-Destillat kontinuierlich im Phasenscheider getrennt, Butanol zurückgeführt und Wasser ausgeschleust. Die Säurezahl des Reaktionsaustrages betrug abzüglich der eingesetzten Schwefelsäure ca. 10 mg KOH/g. Danach wurden 2,2 Äquivalente NaOH bezogen auf eingesetzte Schwefelsäure zugesetzt und dann das überschüssige Butanol bei ca. 120 mbar bis zu einer Sumpftemperatur von 120°C abdestilliert. Der resultierende Rückstand wurde an einem Sambayverdampfer in Destillat (1419 g) und Hochsieder bei 1 mbar und 200°C aufgetrennt. Dieses Destillat wurde dann über eine 75 cm Füllkörperkolonne fraktioniert destilliert (ca. 5 mbar), wobei 1,4- und 1,2-Cyclohexandiole vollständig von Vorfraktionen von Hydroxycapronsäurebutylesterfraktionen, im folgenden Estergemisch bezeichnet, abgetrennt waren.

119 g des Estergemischs (67 % Hydroxycapronsäurebutylester, 10 % Bernsteinsäuredibutylester, 5 % Glutarsäuredibutylester sowie eine Vielzahl anderer Produkte) wurden in einen Destillationskolben mit 0,05 g 1,6-Hexandiol und 1,2 g Titanat versetzt. Bei 200 mbar/150 - 180°C Sumpftemperatur wurde über eine 1 m Drehbandkolonne ca.32 g bei einer Kopftemperatur von 75°C überwiegend Butanol abdestilliert. Nach Reduktion des Drucks auf 20 mbar und Steigerung der Sumpftemperatur auf 200 - 300°C destillierten bei Kopftemperaturen bis 122°C ca. 60 g ab. Davon wurden 50 g an einer 1 m Drehbandkolonne bei 20 mbar fraktioniert destilliert und Caprolacton in einer Reinheit von 99,7 % erhalten.

## Patentansprüche

1. Verfahren zur Herstellung von 1,6-Hexandiol und ε-Caprolacton aus einem Adipinsäure, 6-Hydroxycapronsäure und geringe Mengen 1,4-Cyclohexandiole enthaltenden Carbonsäuregemisch, das als Nebenprodukt der Oxidation von Cyclohexan zu Cyclohexanon/Cyclohexanol mit Sauerstoff oder Sauerstoff enthaltenden Gasen durch Wasserextraktion des Reaktionsgemisches erhalten wird, durch Veresterung und Hydrierung eines Teilstroms zu Hexandiol und Cyclisierung von 6-Hydroxycapronsäureester zu Caprolacton, dadurch gekennzeichnet, daß man
a) die in dem wäßrigen Reaktionsgemisch enthaltenen Mono- und Dicarbonsäuren mit einem niedermolekularen Alkohol zu den entsprechenden Carbonsäureestern umsetzt,
b) das erhaltene Veresterungsgemisch in einer ersten Destillationsstufe von überschüssigem Alkohol und Leichtsiedern befreit,
c) aus dem Sumpfprodukt in einer zweiten Destillationsstufe eine Auftrennung in eine von 1,4-Cyclohexandiolen im wesentlichen freie Esterfraktion und eine zumindest den größeren Teil der 1,4-Cyclohexandiole enthaltende Fraktion durchführt,
d) von der Esterfraktion in einer dritten Destillationsstufe zumindest teilweise einen im wesentlichen 6-Hydroxycapronsäureester enthaltenden Strom abtrennt,
e) die Esterfraktion aus (d), aus der zumindest teilweise 6-Hydroxycapronsäureester entfernt wurde, katalytisch hydriert und durch Destillation des Hydrierproduktes in an sich bekannter Weise 1,6-Hexandiol gewinnt und
f) den im wesentlichen 6-Hydroxycapronsäureester enthaltenden Strom auf Temperaturen über 200°C bei vermindertem Druck erhitzt, wodurch 6-Hydroxycapronsäureester zu Caprolacton cyclisiert wird und aus dem Cyclisierungsprodukt durch Destillation reines ε-Caprolacton gewinnt.

2. Verfahren gemäß Anspruch 1, dadurch gekennzeichnet, daß man das Carbonsäuregemisch vor der Veresterung entwässert.

3. Verfahren gemäß Anspruch 1, dadurch gekennzeichnet, daß man die Veresterung mit Alkanolen mit 1 bis 3 Kohlenstoffatomen durchführt.

4. Verfahren gemäß Anspruch 1, dadurch gekennzeichnet, daß man die Veresterung mit Alkanolen mit 4 bis 10 Kohlenstoffatomen durchführt.

5. Verfahren gemäß Anspruch 1, dadurch gekennzeichnet, daß man die Veresterung mit Methanol durchführt und in der Destillationsstufe (c) eine im wesentlichen von 1,4-Cyclohexandiolen freie Carbonsäuremethylester-Fraktion am Kopf der Kolonne und eine die Hochsieder und die 1,4-Cyclohexandiole enthaltende Sumpffraktion gewinnt und die Carbonsäuremethylesterfraktion in die dritte Destillationsstufe (d) überführt.

6. Verfahren gemäß Anspruch 1, dadurch gekennzeichnet, daß man die Veresterung mit n- oder i-Butanol durchführt und in der Destillationsstufe (c) die 1,4-Cyclohexandiole mit den Leichtsiedern über Kopf abtrennt und die Carbonsäurebuthylester als Seitenstrom oder als diese enthaltenden Sumpf gewinnt und in die dritte Destillationsstufe (d) überführt.

7. Verfahren gemäß Anspruch 1, dadurch gekennzeichnet, daß man die Destillationsstufen (c) und (d) in einer einzigen Kolonne durchführt.

8. Verfahren gemäß Anspruch 7, dadurch gekennzeichnet, daß man im Falle der Veresterung mit Methanol in einem oberen Seitenabzug eine im wesentlichen Dicarbonsäuremethylester enthaltende Fraktion, als unteren Seitenabzug eine im wesentlichen 6-Hydroxycapronsäuremethylesterfraktion und als Sumpfprodukt eine die 1,4-Cyclohexandiole enthaltende Fraktion abtrennt.

9. Verfahren gemäß Anspruch 7, dadurch gekennzeichnet, daß man im Falle der Veresterung mit n- oder i-Butanol in einem oberen Seitenabzug eine im wesentlichen 6-Hydroxycapronsäurebutylester enthaltende Fraktion, als unteren Seitenabzug eine im wesentlichen Dicarbonsäurebutylester enthaltende Fraktion und als Kopfprodukt eine die 1,4-Cyclohexandiole enthaltende Fraktion gewinnt.

10. Verfahren gemäß Anspruch 1, dadurch gekennzeichnet, daß man das Sumpfprodukt der Stufe (c) zumindest teilweise einer erneuten Veresterung unter weiterem Zusatz des niedermolekularen Alkohols und eines Veresterungskatalysators unterwirft und in einer getrennten Destillationsstufe analog (b) und (c) auftrennt oder erst nach Abtrennung der 1,4-Cyclohexandiole die erneute Veresterung durchführt und die die Carbonsäureester enthaltende Fraktion in die Hydrierstufe (d) einleitet.

11. Verfahren gemäß Anspruch 1, dadurch gekennzeichnet, daß man zur Hydrierung Katalysatoren verwendet, die als katalytisch aktive Hauptbestandteile Kupfer, Kobalt und/oder Rhenium enthalten.

12. Verfahren nach Anspruch 1, dadurch gekennzeichnet, daß man zur Hydrierung Katalysatoren verwendet, die in der oxidischen Form die Zusammensetzung CuₐAl_{b}Zr_{c}Mn_{d}Oₓ besitzen, wobei a > 0, b > 0, c ≥ 0, d > 0, a > b/2, b > a/4, a > c und a > d gilt und x die zur Wahrung der Elektroneutralität pro Formeleinheit erforderliche Anzahl von Sauerstoffionen bezeichnet.

13. Abwandlung des Verfahrens gemäß Anspruch 1, dadurch gekennzeichnet, daß man die Hydrierung der Stufe (e) unterläßt und aus der Esterfraktion der Stufe (d) Adipinsäurediester gewinnt.

14. Abwandlung des Verfahrens gemäß Anspruch 1, dadurch gekennzeichnet, daß man für eine vereinfachte Herstellung von Caprolacton für die Veresterung in Stufe (a) einen Alkohol verwendet, der höher als Caprolacton siedet und daß man die Umsetzung, ohne Isolierung der Adipindiesterfraktion, ansatzweise in einer Eintopfreaktion in Gegenwart eines Veresterungskatalysators durchführt, wobei die Umsetzungen der Stufen (b), (c) und (f) im gleichen Ansatz erfolgen, und die hochsiedenden Adipinsäurediester im Sumpf verbleiben und Caprolacton als Destillat gewonnen wird.

## Claims

1. A process for preparing 1,6-hexanediol and ε-caprolactone from a carboxylic acid mixture comprising adipic acid, 6-hydroxycaproic acid and small amounts of 1,4-cyclohexanediols which is obtained as a by-product in the oxidation of cyclohexane to cyclohexanone/cyclohexanol using oxygen or oxygen-containing gases by water extraction of the reaction mixture, by esterifying and hydrogenating a substream to give hexanediol and cyclizing 6-hydroxycaproic esters to give caprolactone, wherein
a) the monocarboxylic and dicarboxylic acids present in the aqueous reaction mixture are reacted with a low molecular weight alcohol to give the corresponding carboxylic esters,
b) the resulting esterification mixture is freed of excess alcohol and low boilers in a first distillation stage,
c) the bottoms are fractionated in a second distillation stage to give an ester fraction essentially free of 1,4-cyclohexanediols and a fraction comprising at least the major part of the 1,4-cyclohexanediols,
d) a stream containing essentially 6-hydroxycaproic esters is separated at least partially from the ester fraction in a third distillation stage,
e) the ester fraction from (d), from which the 6-hydroxycaproic esters have been removed at least partially, is catalytically hydrogenated and 1,6-hexanediol is isolated by distillation of the hydrogenation product in a manner known per se and
f) the stream containing essentially 6-hydroxycaproic esters is heated to above 200°C under reduced pressure, thereby cyclizing 6-hydroxycaproic esters to give caprolactone, and pure ε-caprolactone is isolated from the cyclization product by distillation.

2. A process as claimed in claim 1, wherein the carboxylic acid mixture is dewatered prior to the esterification.

3. A process as claimed in claim 1, wherein the esterification is carried out using alkanols having from 1 to 3 carbon atoms.

4. A process as claimed in claim 1, wherein the esterification is carried out using alkanols having from 4 to 10 carbon atoms.

5. A process as claimed in claim 1, wherein the esterification is carried out using methanol and, in the distillation stage (c), a methyl carboxylate fraction essentially free of 1,4-cyclohexanediols is obtained at the top of the column and a fraction comprising the high boilers and the 1,4-cyclohexanediols is obtained as bottoms, and the methyl carboxylate fraction is transferred to the third distillation stage (d).

6. A process as claimed in claim 1, wherein the esterification is carried out using n- or i-butanol and, in the distillation stage (c), the 1,4-cyclohexanediols are separated off at the top together with the low boilers and the butyl carboxylates are obtained as a side stream or as bottoms comprising these and are transferred to the third distillation stage (d).

7. A process as claimed in claim 1, wherein the distillation stages (c) and (d) are carried out in a single column.

8. A process as claimed in claim 7, wherein, in the case of the esterification using methanol, a fraction containing essentially methyl dicarboxylate is separated off at an upper side offtake, a fraction consisting essentially of methyl 6-hydroxycaproate is separated off as a lower side stream and a fraction comprising the 1,4-cyclohexanediols is separated off as bottoms.

9. A process as claimed in claim 7, wherein, in the case of the esterification using n- or i-butanol, a fraction containing essentially butyl 6-hydroxycaproate is obtained at an upper side offtake, a fraction containing essentially butyl dicarboxylate is obtained as a lower side stream and a fraction comprising the 1,4-cyclohexanediols is obtained as top product.

10. A process as claimed in claim 1, wherein the bottoms from stage (c) are subjected at least partially to renewed esterification with further addition of the low molecular weight alcohol and an esterification catalyst and, in a separate distillation stage, is fractionated by a method similar to (b) and (c), or the renewed esterification is carried out only after separating off the 1,4-cyclohexanediols, and the fraction comprising the carboxylic esters is introduced into the hydrogenation stage (d).

11. A process as claimed in claim 1, wherein the hydrogenation is carried out using catalysts which comprise copper, cobalt and/or rhenium as catalytically active main constituents.

12. A process as claimed in claim 1, wherein the hydrogenation is carried out using catalysts which, in the oxidic form, have the composition CuₐAl_{b}Zr_{c}Mn_{d}Oₓ, where a > 0, b > 0, c ≥ 0, d > 0, a > b/2, b > a/4, a > c and a > d and x corresponds to the number of oxygen ions required per formula unit to achieve electrical neutrality.

13. A modification of the process as claimed in claim 1 in which the hydrogenation of stage (e) is omitted and adipic diesters are isolated from the ester fraction from stage (d).

14. A modification of the process as claimed in claim 1 in which, for a simplified preparation of caprolactone, the esterification of stage (a) is carried out using an alcohol having a boiling point higher than that of caprolactone and the reaction is carried out batchwise, without isolation of the adipic diester fraction, in a single-vessel reaction in the presence of an esterification catalyst, where the reactions of stages (b), (c) and (f) are carried out in the same batch, and the high-boiling adipic diesters remain in the bottom and caprolactone is isolated as distillate.

## Revendications

1. Procédé de préparation de 1,6-hexanediol et d'ε-caprolactone à partir d'un mélange d'acides carboxyliques contenant de l'acide adipique, de l'acide 6-hydroxycaproïque et de faibles quantités de 1,4-cyclohexanediols, mélange qui est obtenu en tant que sous-produit de l'oxydation du cyclohexane en cyclohexanone/cyclohexanol avec de l'oxygène ou des gaz contenant de l'oxygène, par extraction à l'eau du mélange réactionnel, par estérification et hydrogénation d'un courant partiel en hexanediol et cyclisation d'un ester de l'acide 6-hydroxycaproïque en caprolactone, caractérisé en ce que
a) on fait réagir les acides mono- et dicarboxyliques contenus dans le mélange réactionnel aqueux avec un alcool à faible masse moléculaire pour obtenir les esters d'acides carboxyliques correspondants,
b) dans une première étape de distillation, on débarrasse de l'alcool en excès et des composés à bas point d'ébullition le mélange d'estérification obtenu,
c) dans une deuxième étape de distillation, on sépare le produit de fond en une fraction ester pour l'essentiel exempte de 1,4-cyclohexanediols et une fraction contenant au moins la plus grande partie des 1,4-cyclohexane-diols,
d) dans une troisième étape de distillation, on sépare au moins partiellement de la fraction ester le courant contenant l'ester de l'acide 6-hydroxycaproïque,
e) on soumet à une hydrogénation catalytique la fraction ester obtenue en (d), dont on a au moins partiellement éliminé l'ester de l'acide 6-hydroxy-caproïque, et, par distillation du produit de l'hydrogénation, on récupère d'une manière connue en soi le 1,6-hexanediol, et
f) on chauffe à des températures supérieures à 200°C sous pression réduite le courant contenant pour l'essentiel l'ester de l'acide 6-hydroxy-caproïque, ce en conséquence de quoi l'ester de l'acide 6-hydroxycaproïque est cyclisé en caprolactone, et, par distillation, on récupère du produit de la cyclisation de l'ε-caprolactone pure.

2. Procédé selon la revendication 1, caractérisé en ce que le mélange d'acides carboxyliques est déshydraté avant l'estérification.

3. Procédé selon la revendication 1, caractérisé en ce que l'estérification est mise en oeuvre avec des alcanols ayant de 1 à 3 atomes de carbone.

4. Procédé selon la revendication 1, caractérisé en ce que l'estérification est mise en oeuvre avec des alcanols ayant de 4 à 10 atomes de carbone.

5. Procédé selon la revendication 1, caractérisé en ce que l'estérification est mise en oeuvre avec du méthanol, et, dans l'étape de distillation (c), on obtient en tête de la colonne une fraction d'esters méthyliques d'acides carboxyliques pour ainsi dire exempte de 1,4-cyclohexanediols, et une fraction de fond contenant les produits à haut point d'ébullition et les 1,4-cyclohexanediols, la fraction esters méthyliques d'acides carboxyliques étant envoyée dans la troisième étape de distillation (d).

6. Procédé selon la revendication 1, caractérisé en ce que l'estérification est mise en oeuvre avec du n- ou de l'isobutanol, et, dans l'étape de distillation (c), on sépare en tête les 1,4-cyclohexanediols avec les composés à bas point d'ébullition, et on récupère les esters butyliques des acides carboxyliques sous forme d'un courant latéral ou d'un fond les contenant, et on les envoie dans la troisième étape de distillation (d).

7. Procédé selon la revendication 1, caractérisé en ce qu'on met en oeuvre les étapes de distillation (c) et (d) dans une colonne unique.

8. Procédé selon la revendication 7, caractérisé en ce que, dans le cas d'une estérification au méthanol, on sépare dans un soutirage latéral supérieur une fraction contenant pour l'essentiel des esters méthyliques d'acides dicarboxyliques, en tant que soutirage latéral inférieur une fraction contenant pour l'essentiel l'ester méthylique de l'acide 6-hydroxycaproïque, et en tant que produit de fond une fraction contenant les 1,4-cyclohexanediols.

9. Procédé selon la revendication 7, caractérisé en ce que, dans le cas d'une estérification au n- ou à l'isobutanol, on obtient dans un soutirage latéral supérieur une fraction contenant pour l'essentiel l'ester butylique de l'acide 6-hydroxycaproïque, en tant que soutirage latéral inférieur une fraction contenant pour l'essentiel des esters butyliques d'acides dicarboxyliques, et en tant que produit de tête une fraction contenant les 1,4-cyclohexanediols.

10. Procédé selon la revendication 1, caractérisé en ce qu'on soumet le produit de fond de l'étape (c) au moins partiellement à une nouvelle estérification, en ajoutant encore l'alcool à faible masse moléculaire et un catalyseur d'estérification, et, dans une étape de distillation distincte, on le sépare d'une manière analogue à (b) et (c), ou encore ce n'est qu'après séparation des 1,4-cyclohexanediols que l'on procède à la nouvelle estérification et que l'on introduit dans l'étape d'hydrogénation (d) la fraction contenant les esters d'acides carboxyliques.

11. Procédé selon la revendication 1, caractérisé en ce qu'on utilise pour l'hydrogénation des catalyseurs qui en tant que constituants principaux catalytiquement actifs contiennent du cuivre, du cobalt et/ou du rhénium.

12. Procédé selon la revendication 1, caractérisé en ce qu'on utilise pour l'hydrogénation des catalyseurs qui, sous forme d'oxydes, ont la composition CuₐAl_{b}Zr_{c}Mm_{d}Oₓ, où a > 0, b > 0, c ≥ 0, d > 0, a > b/2, b > a/4, a > c et a > d, et x désigne le nombre d'ions oxygène nécessaires au maintien de la neutralité électronique par formule unitaire.

13. Variante du procédé selon la revendication 1, caractérisée en ce qu'on omet l'hydrogénation de l'étape (e), et on obtient un diester de l'acide adipique à partir de la fraction ester de l'étape (d).

14. Variante du procédé selon la revendication 1, caractérisée en ce que, pour une préparation simplifiée de la caprolactone, on utilise pour l'estérification de l'étape (a) un alcool dont le point d'ébullition est plus élevé que celui de la caprolactone, et en ce qu'on met en oeuvre la réaction, sans isolement de la fraction diester de l'acide adipique, par portions dans une réaction en une cuve en présence d'un catalyseur d'estérification, les réactions des étapes (b), (c) et (f) étant réalisées dans la même masse réactionnelle, et les diesters de l'acide adipique à haut point d'ébullition restent dans le fond, la caprolactone étant récupérée sous forme d'un distillat.
